# EUROPEAN PATENT APPLICATION

(11) **EP 4 285 823 A2**
(43) Date of publication of application: **06.12.2023**
(21) Application number: 23174968.0
(22) Date of filing: 23.05.2023
(51) Int. Cl.: A61B 5/361

(54) **CARDIAC MONITORING AND MANAGEMENT SYSTEM**

(30) Priority: 03.06.2022 US 202263348959 P; 09.01.2023 US 202318094810
(71) Applicant: Apple Inc., Cupertino, CA 95014 (US)
(72) Inventor: MARTINEZ, Ainara Garde, Cupertino, 95014 (US); BROUSE, Christopher J., Cupertino, 95014 (US); WAYDO, Stephen J., Cupertino, 95014 (US)
(74) Representative: Zacco Denmark A/S

(57) **Abstract**

Embodiments are directed to systems and methods for determining a cardiac burden of a user. Methods include collecting a series of cardiac measurements at a first set of time points using one or more sensors and determining a set of measured data points. Each measured data point can include a time value from the first set of time points and a classification comprising one of an occurrence of a qualified cardiac event or absence of the qualified cardiac event. Methods include generating a set of additional data points each including a time value from the second set of time points and a classification of one of the occurrence of the qualified cardiac event or the absence of the qualified cardiac event. Methods include determining a value of the cardiac burden for a predefined period of time using the set of measured data points and the set of additional data points.

## Description

### FIELD

The described embodiments relate generally to systems and methods for monitoring cardiac parameters of a user and more particularly to systems and methods for determining a cardiac burden of a user.

### BACKGROUND

Wearable electronic devices are increasingly incorporating sensing systems that include one or more sensors that measure physiological parameters of a user. These sensors may be able to collect physiological information about a user over longer periods of time such as multiple hours, days and weeks. There may be instances, however, where a sensor is unable to collect this physiological information, such as when a user is not actively wearing the sensor. In other instances, motion associated with a user's activity may interfere with collecting physiological information. For example, if a user is running, exercising, or performing other similar activities, the higher levels of movement from these activities may introduce a significant amount of noise into measured signals, may temporarily displace the sensor relative to the user, or may otherwise interfere with the collection of physiological information. Accordingly, in some cases, physiological information may be collected sporadically over one or more periods of times, which may make it difficult to consistently track physiological information for a user.

### SUMMARY

Embodiments are directed to methods for determining a cardiac burden of a user. The methods can include collecting a series of cardiac measurements at a first set of time points using one or more sensors and determining a set of measured data points. Each measured data point can include a time value from the first set of time points and a classification including one of an occurrence of a qualified cardiac event or absence of the qualified cardiac event. The classification can be selected using a cardiac measurement of the series of cardiac measurements associated with a corresponding time window from the first set of time points. The methods can include generating a set of additional data points associated with a second set of time points different from the first set of time points. Each additional data point can include a time value from the second set of time points and a classification of one of the occurrence of the qualified cardiac event or the absence of the qualified cardiac event. The classification can be selected using one or more measured data points of the set of measured data points. The methods can include determining a value of the cardiac burden for a predefined period of time using the set of measured data points and the set of additional data points and outputting the value of the cardiac burden.

Embodiments are also directed to devices for measuring cardiac parameters of a user. The devices can include one or more sensors configured to collect cardiac measurements of the user and a processor. The processor can be configured to operate in a first mode to cause the one or more sensors to collect a first set of cardiac measurements and input each cardiac measurement of the first set of cardiac measurements as an input cardiac measurement into a classification model to produce a first set of outputs, where a first threshold is applied to the first set of outputs for identifying whether the input cardiac measurement is a qualified cardiac event. In response to the first set of outputs meeting a first set of criteria, the processor can be configured to output an indication of an occurrence of the qualified cardiac event to the user. The processor configured to operate in a second mode to collect a second set of cardiac measurements and input each of the second set of cardiac measurements as an input cardiac measurement into the classification model to produce a second set of outputs, where a second threshold different than the first threshold is applied to the second set of outputs for identifying whether the input cardiac measurement is the qualified cardiac event. The processor can be configured to determine a value of a cardiac burden for predefined period of time based on the second set of outputs and output the value of the cardiac burden.

Embodiments are further directed to a system for measuring cardiac parameters of a user. The system can include one or more sensors configured to collect cardiac measurements of the user and a processer configured to collect a series of cardiac measurements at a first set of time points using the one or more sensors. The processor can be configured to determine a set of measured data points, where each measured data point includes a time value from the first set of time points and a classification comprising one of an occurrence of a qualified cardiac event or absence of the qualified cardiac event. The classification can be selected using a cardiac measurement of the series of cardiac measurements associated with the time value. The processor can be configured to generate a set of additional data points associated with a second set of time points different from the first set of time points, where each additional data point includes a time value from the second set of time points and a classification of one of the occurrence of the qualified cardiac event or the absence of the qualified cardiac event. The classification can be selected using one or more measured data points of the set of measured data points. The processor can be configured to determine a value of a cardiac burden for a predefined period of time using the set of measured data points and the set of additional data points and output the value of the cardiac burden.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows a system diagram for an electronic device that can be used to perform the cardiac monitoring and management processes described herein;
FIG. 2 shows a process flow for determining a cardiac burden of a user;
FIG. 3 shows an example of a process for generating a set of additional cardiac data points from a set of measured cardiac data points;
FIG. 4 shows a process flow for determining cardiac burdens for different periods of time;
FIGs. 5A-5C show example outputs of cardiac burdens for different periods of times;
FIG. 6 shows a process flow for operating a device in different cardiac monitoring modes; and
FIG. 7 is an example block diagram of a cardiac monitoring system.

It should be understood that the proportions and dimensions (either relative or absolute) of the various features and elements (and collections and groupings thereof) and the boundaries, separations, and positional relationships presented therebetween, are provided in the accompanying figures merely to facilitate an understanding of the various embodiments described herein and, accordingly, may not necessarily be presented or illustrated to scale, and are not intended to indicate any preference or requirement for an illustrated embodiment to the exclusion of embodiments described with reference thereto.

### DETAILED DESCRIPTION

Reference will now be made in detail to representative embodiments illustrated in the accompanying drawings. It should be understood that the following descriptions are not intended to limit the embodiments to one preferred embodiment. To the contrary, it is intended to cover alternatives, modifications, and equivalents as can be included within the spirit and scope of the described embodiments as defined by the appended claims.

Embodiments disclosed herein are directed to cardiac monitoring systems that can be configured to monitor cardiac parameters of a user, specifically a cardiac burden associated with a given type of cardiac event. As used herein, the terms "cardiac burden" or just "burden", expressed as a percentage, refers to the relative amount of a period of time that a user is experiencing a particular type of cardiac event. The type of cardiac event may be any detectable irregularity in a user's heartbeats or heart rhythms, such as atrial fibrillation, atrial flutter, premature ventricular contractions, or premature atrial contractions. While examples of the cardiac monitoring systems described herein are used to measure an atrial fibrillation burden (i.e., the cardiac burden when atrial fibrillation is the type of cardiac event), the principles described here may be applied to any suitable type of cardiac event and its associated cardiac burden. Furthermore, some embodiments include measuring multiple different cardiac burdens for different types of cardiac events.

To measure a cardiac burden, the systems described herein include one or more devices that have one or more sensors configured to collect a cardiac measurement, which represents information about a user's heartbeats (e.g., timing information and/or morphology thereof) measured over a period of time (e.g., ten second, thirty seconds, or sixty seconds) referred to herein as the "measurement duration." The system may attempt to collect multiple cardiac measurements over time, and a set of these cardiac measurements may be analyzed to determine a cardiac burden. The systems and methods described herein may collect these cardiac measurements and calculate an associated cardiac burden over sustained stretches of time such as multiple hours, days, weeks, months and so on. Additionally, the systems and methods described herein may analyze and identity windows of times when a cardiac event is more common for an individual user.

The one or more sensors may include any sensors capable of collecting a cardiac measurement. For example, in some instances the one or more sensors can include optical sensors such as a photoplethysmography (PPG) sensors. Additionally or alternatively, the one or more sensors includes electrical sensors such as an electrocardiogram (ECG) sensors. These sensors incorporated into one or more wearable devices such as a smart watch, band, ring or any other suitable wearable device. It should be appreciated that the cardiac monitoring systems may collect cardiac measurements from multiple different devices, each including a respective set of sensors. Additionally, these devices may include one or more additional sensors that may be used to measure motion (e.g., an accelerometer, gyroscope, or the like) and/or other physiological signals of a user, which may facilitate or supplement the collection of cardiac measurements.

While it may be desirable to obtain several cardiac measurements for the purpose of calculating a cardiac burden, there may be certain stretches of time during which the cardiac monitoring systems are unable to obtain a usable cardiac measurement. For example, the accuracy of a cardiac measurements made by the sensors may be affected by movement. If the level of movement is high enough (e.g., if a user is engaged in vigorous exercise), the cardiac sensors may not be able to obtain cardiac measurements with sufficient accuracy to determine whether a user is experiencing a particular cardiac event.

Accordingly, in some variations the systems described herein may attempt to collect a cardiac measurement for each of a set of predefined time intervals. In some of these instances, the system may only take a cardiac measurement during a given time interval if a certain set of criteria is satisfied (which may represent a determination that an accurate cardiac measurement can be achieved). For example, the system may determine a motion state associated with a given sensor (e.g., using an accelerometer), and the set of criteria includes determining that the motion state for that sensor is below a threshold (e.g., a motion threshold). In these instances, a sensor may not make a measurement unless there is low enough motion for the sensor to achieve an accurate measurement. In this way, it is possible that the system will not collect a cardiac measurement during a given time interval because the set of criteria is not satisfied (which would be considered an unsuccessful attempt).

In some instances, the system may collect a cardiac measurement during a time interval but may not use that cardiac measurement for the purpose of determining a cardiac burden. For example, if the system determined that the user's movement was above a threshold (e.g., another motion threshold that may be the same as different from the motion threshold used to determine whether to collect a measurement) during collection of the cardiac measurement, it may discard data from that measurement. In other instances, the system may determine that a given cardiac measurement fails to meet one or more quality metrics and may discard the data from cardiac measurement. It should be appreciated that although a collected cardiac measurement may be discarded for the purpose of calculating a particular cardiac burden, the system may keep that data for other purposes (e.g., to calculate another physiological parameter of the user). When a cardiac measurement is collected but discarded for the purpose of calculating a cardiac burden, this is also considered an unsuccessful attempt. Accordingly, the system may collect cardiac measurements during some time intervals, but not others.

Each cardiac measurement is associated with a "time point," which is a set of time values that represent a particular point or period of time. Time point may be used to refer and/or label the timing of the cardiac measurements that were collected over a given stretch of time. In some instances, the time point may include a single time value used to represent a single moment in time (e.g., the time at which the cardiac measurement was initiated, the time at which the cardiac measurement was terminated, the beginning of a time interval associated with the cardiac measurement, or the like). In this way, although the cardiac measurement may be taken over a measurement duration as described above, data associated with this measurement may be linked to a single time value. In other instances, the time point may include multiple time values that define a period of time (e.g., the beginning and end of the cardiac measurement, the beginning and end of a time interval that included the cardiac measurement). The timing information of the cardiac measurements may be used in calculating a cardiac burden as discussed below.

For example, system may analyze a series of cardiac measurements to identify whether a user was having a particular type of cardiac event at each of the time points associated with the cardiac measurements. Specifically, there may be a set of qualification criteria for a given type of cardiac event required for the system to consider a cardiac measurement (and its associated time point) to be considered to be an instance of the cardiac event. As a result, each cardiac measurement is either considered to be an occurrence of a qualified cardiac event (i.e., if it meets the qualification criteria for that type of cardiac event) and can be classified accordingly. In some instances, this classification is achieved by inputting data from a cardiac measurement into a classification model such as a neural network or other machine learning model. The classification model may output a confidence value indicative of the likelihood that the cardiac measurement was representative of an occurrence of the cardiac event. If this confidence value is exceeds a threshold (e.g., a confidence threshold) that serves as the qualification criteria, the cardiac measurement may be classified as an occurrence of the qualified cardiac event.

As an illustrative example, when the type of cardiac event is atrial fibrillation, a classification model may output a confidence value that is indicative of the likelihood that a user was experiencing atrial fibrillation during a given cardiac measurement. In some of these instances, the cardiac measurement may include a tachogram, and the classification model may receive the tachogram as an input. The classification model may be trained using a data set that includes both tachograms associated with atrial fibrillation and tachograms that are not associated with atrial fibrillation (e.g., sinus rhythm). The threshold (e.g., the confidence threshold mentioned above) applied to the output of the classification model may be selected to achieve a particular level of sensitivity or specificity. Collectively, the classified cardiac measurements may be analyzed to form a set of measured data points, each including a time value associated with the time point for a given cardiac measurement and the classification of whether cardiac measurement was an occurrence or absence of the qualified cardiac event.

As discussed herein, the system may not be able to collect a cardiac measurement during each of the time intervals, for example due to unsuccessful attempts as discussed above or a user not wearing a device including the sensor(s), and so on. Accordingly, the set of measured data points may include data that is sporadic over a period of time. While the system controls the sensor to attempt a cardiac measurement at a regular interval (e.g., every five minutes, every ten minutes, every fifteen minutes, every half hour, or the like) over the course of the day, it may only successfully collect cardiac measurements and generate corresponding data points for only some of those attempts. Accordingly, there may be time intervals during which no measured data points exist, which may make it difficult for the system to calculate a cardiac burden for a given stretch of time.

Accordingly, in some instances the systems described herein are configured to generate a set of additional data points that fill in some or all of the gaps from the set of measured data points. For example, the system may use the measured data points to estimate whether a user was experiencing a cardiac event at certain time points where the system was not able to successfully obtain a cardiac measurement (e.g., due to noise). The set of additional data points may each include a time value that represents a time point for which the data point is being generated. Each additional data point may also include a classification of whether a qualified cardiac event occurred or did not occur at the corresponding time point.

The additional data points are generated using the measured data points. In this way, although the system was unable to obtain a successful cardiac measurement in a given time interval, it still may be able to estimate whether a user was experiencing a type of cardiac event during this interval. For example, in some cases, the classification for each additional data point is based on a classification of the closest measured data point. For example, each additional data point may be assigned the same classification as the nearest data point. In some instances, the system may require certain criteria to be met before generating certain additional data points, such as described in more detail below. Accordingly, the system may generate data points for time points that did not have a corresponding cardiac measurement. In this regard, the system can generate a data set that covers a larger amount of time, which may help characterize a user's cardiac burden over long time periods and/or how a user's cardiac burden changes over a time period.

The systems described herein may use the measured data points and the additional data points that are generated from the measured data points to determine a cardiac burden for the user. This data may be used to calculate the cardiac burden over different periods of time. In some instances, this is a continuous period of time (e.g., an hour or set of hours, a day or set of days, a week or set of weeks), such that the system provides a value of cardiac burden for that period of time. In this way, the system may provide a cardiac burden for a particular hour or set of hours, day or set of days, week or set of weeks, or the like. This may provide information on how a user's cardiac function changes over time, such as by showing how the cardiac burden changes every day over the span of a week or month.

In other instances, the period of time is a recurring time window (e.g., a particular hour or set of hours, day or set of days, or the like), which includes multiple noncontiguous periods of time. As an example, the recurring time window may include a set of Mondays, such that the system provides a cardiac burden for a certain number of Mondays. These recurring time windows may allow a user to see how their cardiac burden varies as a function of time of day (e.g., whether it is generally better in the morning compared to the evening), days of the week (e.g., whether it is better on Mondays as compared to Fridays), or the like.

In some cases, the cardiac burden data can also include other user physiological and/or lifestyle data. For example, the system may collect data on a user's stress level, sleep, diet, exercise, or any other suitable parameters. This data may be correlated and/or output with the cardiac burden data. For example, if a use routinely experiences the highest cardiac burden on a Monday, the system may correlate this to the person also having the highest stress levels on Monday.

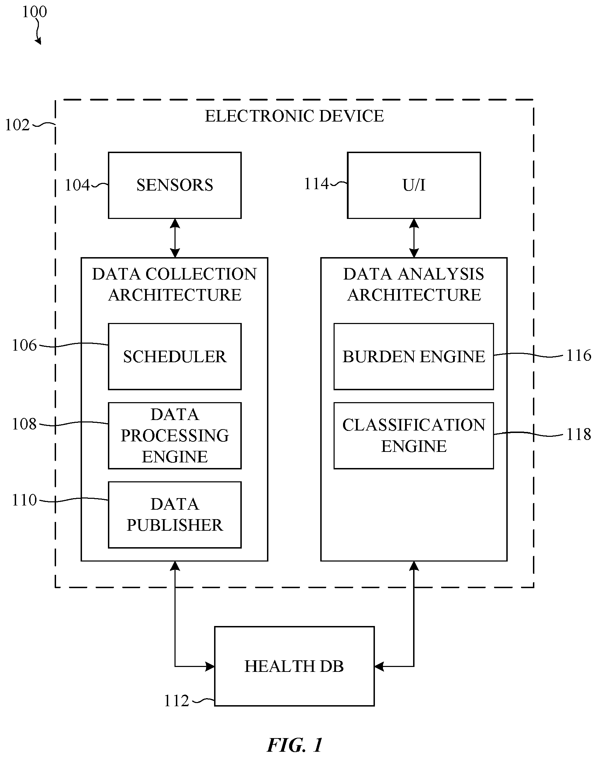

These and other embodiments are discussed below with reference to FIGs. 1-7. However, those skilled in the art will readily appreciate that the detailed description given herein with respect to these Figures is for explanatory purposes only and should not be construed as limiting.

FIG. 1 shows a system 100 for an electronic device that can be used to perform the cardiac monitoring and management processes described herein. The system 100 can include one or more electronic devices 102 (one or which is shown for clarity, though multiple separate devices may be possible). Each electronic device 102 may include all or some of the architecture of the system. For example, in some cases a first electronic device 102 may include one or more sensors 104 that are operated to measure physiological parameters of a user and a second electronic device may analyze the measured data to determine one or more cardiac parameters such as a cardiac burden. In other cases, a single electronic device 102 may include the sensor systems for measuring physiological parameters and system architecture for analyzing the measured data to generate one or more cardiac parameters.

For clarity of explanation, the system 100 is described with respect to a single electronic device, however, the hardware (e.g., sensor systems, processors, etc.) and system architecture (e.g., data analysis software) can be implemented across a variety of electronic devices. For example, the system 100 can include a wearable electronic device 102 which includes one or more sensors 104 that measure physiological parameters (e.g., via cardiac measurements). The wearable electronic device 102 may perform some data analysis function such as digitizing, filtering, saving and/or other data analysis processes described herein. In some cases, the wearable electronic device 102 may store the collected data in a database 112 and/or send the collected data to another electronic device for further processing and analysis. In some cases, other electronic devices 102 can retrieve the measured physiological data from the database 112 and process the data to identify a cardiac event and/or determining a cardiac burden of a user. One or more the electronic devices 102 may output the cardiac metric(s) to a user. The electronic device(s) 102 may output the cardiac metrics in a variety of ways including through a user interface (U/I) 114, such as a display, using audio alerts, through haptic feedback, or any other suitable output mechanism, or combinations thereof.

The sensors 104 can include one or more sensors that are configured to measure cardiac parameters of a user and generate cardiac measurements. In some cases, the sensors 104 can include light-based sensors that emit light toward a user and determine cardiac parameters based on a detecting light that has interacted with the user. The light-based sensors can include light emitters and detectors that operate across various wavelength, a single wavelength or a defined range of wavelengths. The light-based sensors can operate in the green wavelength range (e.g., from about 520 to about 560 nanometer range), the infrared (IR) wavelength range (e.g., in the near-IR range from about 760 to about 1500 nanometers) or in any other suitable wavelength ranges such as those associated with red light and/or blue light. The light-based sensor can be configured as a PPG sensor that measures blood volume changes of a user, which can be used to determine cardiac properties such as the timing of individual heartbeats, heart rate over time, respiration rate, blood oxygenation, and so on.

The sensors 104 can include sensors that are configured to measure electrical cardiac parameters of a user. For example, the sensors 104 can include ECG sensors that generate electrocardiogram data of a user electrical heart activity. The ECG data may be used on its own to generate one or more cardiac metrics such as a cardiac burden and/or analyzed with other sensing data, such as data from PPG sensing. For example, determinations of the occurrence of a qualified event from each of the PPG sensing and the ECG sensing may be compared. If both indicate the occurrence of a cardiac event, the system determines that a qualified cardiac event occurred. If the outputs from the different sensing modalities are conflicting, the system can be configured to select one of the sensing modalities over the other, such as using the output from the ECG sensor, since, in some cases that may be considered more accurate or vice versa. In other cases, the system may compare confidence metrics associated with the different sensing modalities and use the output from the modality with the higher confidence.

The sensors 104 can include motion-based sensing systems such as one or more accelerometers. The accelerometers can measure motion of a wearable device attached to a user and may be configured to measure motion along one or more dimensions. In some cases, the sensors 104 can be configured to measure angular motion, such as through the use of gyroscopes or other suitable systems. Additionally or alternatively, the sensors can include other types of physiological sensors such as temperature sensors, light sensors, sound sensors and so on.

The electronic device 102 may be configured with data collection architecture which can control the sensors 104 to obtain physiological measurements (e.g., cardiac measurements) of a user. The data collection architecture may include a scheduler 106, a data processing engine 108 and a data publisher 110. The data collection architecture may be supported by one or more hardware systems such as processor(s), memory, a power source, as described herein.

The scheduler 106 may be configured to determine when physiological measurements are to be taken and communicate with the sensor to initiate and control the collection these measurements. In some cases, the scheduler 106 can be configured to initiate measurements on defined interval. For example, the scheduler 106 may initiate measurements a set time interval such as every 5 minutes, 10 minutes, 15 minutes, 30 minutes or other suitable intervals which may be shorter or longer. In some cases, the scheduler 106 may control the sensor(s) 104 to attempt a measurement at the start of each time interval, and if successful the measured data is processed and stored as described herein. If a measurement is unsuccessful, the scheduler 106 may take no further action until the next interval. In other cases, a measurement may be initiated at the beginning of an interval, and if unsuccessful, the scheduler may look for other opportunities within the interval until a successful measurement is achieved or the interval ends (at which point the process would move to the next interval).

In other cases, the scheduler 106 may dynamically initiate and control physiological measurements. For example, in response to an unsuccessful measurement attempt at a time point, the scheduler 106 may initiate one or more additional measurements attempts within a time window associated with the time point. For example, the scheduler 106 may be configured to initiate a measurement every 15 minutes, where each 15-minute window is associated with a time point. If a first measurement within a 15-minute window is unsuccessful, the scheduler 106 may initiate one or more additional measurements in an effort to collect a measurement for that time point.

The data processing engine 108 can be configured to receive data from the sensors 104 and processes the data for storage and/or to be used for determining a cardiac burden of a user. The data processing engine 108 may be configured to digitize raw sensor signals, filter and/or otherwise remove noise, up-sample or down-sample data, and/or the like. The data processing engine 108 can process data into a format that is input into other systems such as a classification engine and/or burden engine as described herein. In some cases, the data processing engine 108 can format metadata, for each successful measurement, which can include a time interval of the associated measurement data, sensing modalities (e.g., a type of sensor obtained the measurement data), device identification (e.g., an identifier associated with the specific device used to take the measurement) or any other suitable information about the data.

The data publisher 110 can be configured to receive data from the processing engine and save the data to the database 112. Additionally or alternatively, the data publisher 110 can send data to other systems within the device 102 or other devices for further analysis. The data publisher 110 may use push and/or pull operation to save the data to the health database 112 and/or send the data for analysis. In some cases, the data publisher 110 format and/or generate metadata, which may be used to uniquely identify a data set (e.g., associated with a specific time point), this may be done in addition or as an alternative to processes performed by the data processing engine.

The electronic device 102 may be configured with data analysis architecture which can control process measured data to determine one or more cardiac burdens for a user. The data analysis architecture may include a cardiac burden engine 116 and a classification engine 118. The data analysis architecture may be supported by one or more hardware systems such as processor(s), memory, a power source, as described herein.

The burden engine 116 may be configured to generate one or more cardiac burden measurements, as described herein. The burden engine 116 may retrieve/receive cardiac measurements from the database 112 and/or from data collection architecture and analyze those measurements to determine a cardiac burden of a user. The burden engine 116 may determine how much of a defined period of time that a user is experiencing a cardiac event (e.g., an estimated percentage of the period of time that the cardiac event was occurring). In some cases, the burden engine 116 may determine multiple cardiac burdens, which may each correspond to different periods of time. For example, the burden engine 166 may determine: a hourly burden, which may estimate a user's cardiac burden over a reoccurring hourly time window from each of a number of days (e.g., from 8:00-10:00 AM each day); a reoccurring daily burden, which may estimate a cardiac burden across each of a specific day of the week over a period of weeks (e.g., a percentage of time on Mondays that the user had a cardiac event); a weekly burden, which may estimate how often a user had a cardiac event over the period of a week; or other suitable periods of time.

The burden engine 116 can be configured to determine cardiac burdens for different types of cardiac events. In some cases, the burden engine 116 can be configured to identify any detectable irregularity in a user's heartbeats or heart rhythms, such as atrial fibrillation, atrial flutter, premature ventricular contractions, or premature atrial contractions or other abnormal heart conditions and determine a burden based on the amount that the arrhythmia is occurring over a period of time. The cardiac burden engine 116 may be configured to identify a cardiac event by determining if cardiac measurements meet one or more qualification criteria. This classification can be achieved by inputting data from a cardiac measurement into a classification model such as a neural network or other machine learning model. The classification model may output a confidence value indicative of the likelihood that the cardiac measurement was representative of an occurrence of the cardiac event. If this confidence value is above a confidence threshold that serves as the qualification criteria, the cardiac measurement may be classified as an occurrence of the qualified cardiac event. The burden engine 116 may analyze individual cardiac measurements that are taken at each time point generate a measured data point that includes a time value associated with the cardiac measurement and the classification (e.g., an occurrence of a qualified cardiac event or the absence of a qualified cardiac event). Accordingly, the outputs from the classification model may be used to generate a set of measured data points for the time points in which cardiac measurements were obtained.

The burden engine 116 may generate additional data from the measured cardiac data, for example, using the set of measure data points. These additional data points may be generated for a set of time points that correspond to time intervals during which cardiac measurements were not obtained and may be used along with the measured data points to determine a cardiac burden as described herein.

The burden engine 116 may include a classification model with a first configuration, which is used to identify cardiac event for determining a cardiac burden. In other cases, the classification techniques described herein may be used to classify cardiac measurements for purposes other than burden calculations. In these cases, the burden engine 116 may be configured to apply different criteria to the outputs from the classification process. For example, the classification engine 118 may include the classification model with a second configuration, which is used to detect the occurrence of a cardiac event with different specificity or sensitivity. For example, the classification engine 118 may be used when the device 102 is operating in a first mode that is configured to identify whether a cardiac event is occurring and the burden engine 116 may be used 102 when the device is operating in a second mode that is configured to identify a frequency/amount the cardiac event is occurring. Accordingly, the classification engine 118 may be configured with a classification model that has higher specificity for identifying a cardiac event and the burden engine 116 may be configured with a classification engine that has higher sensitivity for the cardiac event.

In other cases, the burden engine 116 can be configured to adjust a threshold (e.g., a confidence threshold) based on a specific user's data. For example, the burden engine 116 may adjust a sensitivity of the classification based on how a specific user's atrial fibrillation present. For example, some users may have atrial fibrillation with a more regular rhythm and the burden engine 116 may adjust the threshold to increase the sensitivity of the detection.

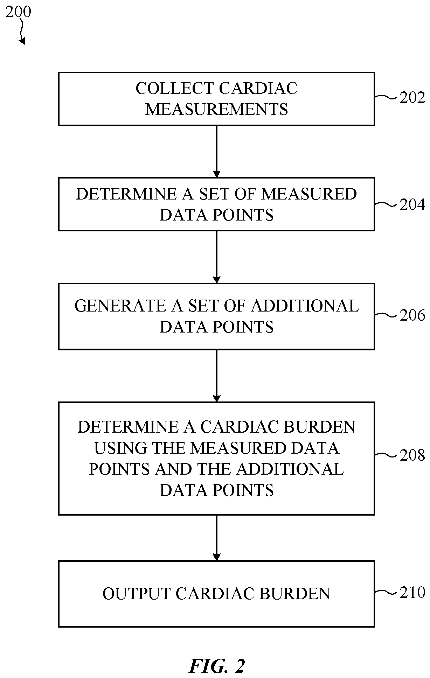

FIG. 2 shows a process 200 for determining a cardiac burden of a user. The process 200 can be performed by the cardiac monitoring systems described herein such as the system 100. The process 200 may be used to determine a cardiac burden of a user for a defined period of time, which may include the periods of time described herein.

At operation 202 the process 200 includes collecting a series of cardiac measurements of a user. The measurements can be collected at a first set of time points using one or more sensors, such as the sensors described herein. The first set of time points can correspond to times that the cardiac measurements were attempted, which may be at a scheduled interval and/or dynamically determined. For example, in some cases a cardiac measurement may be attempted at a defined interval (e.g., every 15 minutes) and each time point of the first set of time points may correspond to an attempted measurement. In some case, the time points may indicate a time the cardiac measurement was attempted. Additionally or alternatively, the time points can indicate a duration of the measurement and/or the defined interval. Accordingly, each time point may be associated with a window of time for the cardiac measurement and/or window corresponding to the measurement interval.

At operation 204 the process 200 can include determining a set of measured data points using successful cardiac measurements taken at various time points. In some cases, each measured data point can correspond to a successful cardiac measurement taken at a time point. Each data point can include a time value that indicates the time point of the corresponding cardiac measurements. For example, each time value may indicate a time that the cardiac measurement was initiated. Additionally or alternatively, a time value may indicate a duration of the cardiac measurement. In other cases, the time value may simply indicate a relative order of successful cardiac measurements and/or an interval of the cardiac measurement.

Each measured data point can also include a classification that indicates whether the associated cardiac measurement was an occurrence or absence of a qualified cardiac event. The classification can be based on analyzing the data from successful cardiac measurements associated with a time point. In some cases, the measured cardiac data may be input into a trained classification model and the classification model outputs an indication of whether a qualified cardiac event occurred or not. For example, the model may include a threshold (such as a confidence threshold as discussed above) or other criteria for classifying measured data as a qualified cardiac event, and classifications that satisfy the threshold are labeled as the occurrence of a qualified cardiac event and classifications that do not satisfy the threshold are labeled as an absence of a qualified cardiac event.

The classification model can be trained with data to identify specific types of cardiac events such as arrhythmias. In one set of examples, the collected data can include heart rate data and the classification model can be configured to identify atrial fibrillation events using the heart rate data. Outputs from the classification model can be used to assign a classification to each measured data point. For example, outputs from the classification model that have a likelihood of atrial fibrillation occurring during a time point above the threshold can be used to assign the corresponding measured data point a classification of the occurrence of a qualified cardiac event (e.g., atrial fibrillation). Outputs from the classification that have a likelihood of atrial fibrillation occurring during a time point below the threshold can be used to assign the corresponding measured data point a classification of the absence of the qualified cardiac event (e.g., bot atrial fibrillation detected). Accordingly, each measured data point can indicate whether the qualified cardiac event was detected during the corresponding time point.

At operation 206 the process 200 can be configured to generate a set of additional data points, where each additional data point is associated with a time point corresponding to an unsuccessful cardiac measurement. The additional data points can be generated to produce additional cardiac data for unsuccessful measurement attempts, which may help develop data sets with enough data to produce significant results. For example, the additional data points may result in data sets that have enough data points to provide more robust statistical metrics for characterizing a cardiac metric such as a cardiac burden. For this reason, the additional data points may allow a cardiac burden to be determined with higher certainty and/or greater time accuracy.

Each additional data point can include a time value that indicates the time point. The additional data points can be generated for time points for which there is not cardiac measurement data. Accordingly, in some cases, each additional data point can correspond to time points corresponding to an unsuccessful cardiac measurement. Accordingly, the time points for the additional data points may be different from the time points for the measured data points. Additionally or alternatively, the additional data points can be generated for time points that have a different interval/ frequency than the attempted cardiac measurements. For example, if a cardiac measurement is attempted at a first time interval (e.g., every 15 minutes) the additional data points can be generated for a second time interval (e.g., for every 5 minutes).

Each additional data point can also include a classification that indicates whether a qualified cardiac event occurred during the associated time point or whether a qualified cardiac event was not detected. The classification for each additional data point can be selected using one or more of the measured data points of the set of measured data points, as described herein. For example, in some cases the additional data point can be assigned the same classification of the closest measured data point. In other cases, each additional data point may be assigned a classification based on more than one measured data point. For example, the predominant classification from the three closest measured data points may be assigned to the additional data points.

At operation 208 the process 200 can include determining a cardiac burden for a predefined period of time. The cardiac burden can indicate an estimated amount of the period of time in which the user experienced the qualified cardiac event. For example, if the system is configured to detect atrial fibrillation, the cardiac burden can indicate an estimated amount of the period of time that the user experienced atrial fibrillation. The predefined period of time may be a continuous period of time or a reoccurring time window, as described previously.

The cardiac burden can be determined from the set of measured data points and the set of additional data points. In some cases, the additional data points can be generated based on the time period for which the cardiac burden is being determined. For example, if the cardiac burden is being generated for a predefined continuous period of time such as a specific week, the system may retrieve cardiac measurements from that weekly period and generated a set of measured data points for the specific week. The system may generate the additional data points based on the predefined period of time. For example, for a weekly burden, the system may generate additional data points at an interval that is specific to the weekly period. For example, the system may generate more additional data point s for shorter periods of time.

In some cases, the system doesn't calculate a burden unless there is a predetermined amount of measured data points in the period of time that is of interest. For example, the system may determine whether the measured data points satisfy a criteria. The criteria may be a minimum amount of data points needed to determine a cardiac burden for a period of time to a predefined accuracy or other quality metric. For shorter periods of time, such as an hourly or daily period of time, the system may collect multiple re-occurring periods of data to meet the criteria. For example, for a re-occurring daily period of time (e.g., Mondays) the system may generate measured data points and additional data points for multiple Mondays before meeting the criteria and generating a cardiac burden from the re-occurring daily time period. If the criteria is met, the system may generate the additional data points. In other cases, the criteria may be based on both the measured data points and the additional data points. In these cases, the system may generate the additional data points and then determine if a minimum data criteria is met.

At operation 210 the process 200 can include outputting the cardiac burden. In some cases, this can include outputting a value for the cardiac burden. The cardiac burden can be output in any suitable format such as on a display including text, videos, animations, or other graphic representations The cardiac burden can be conveyed visually (e.g., using audio feedback). In some cases, the burden can be managed and monitored as part of an application and displayed when requested by the user, for example, in response to the user opening and/or interacting with a specific application. In other cases, the system can automatically output the burden to the user in response to the cardiac burden meeting a defined criteria. For example, the defined criteria may be configured to alert a user if the detected cardiac burden is above a defined value (e.g., over 80% of the period of time).

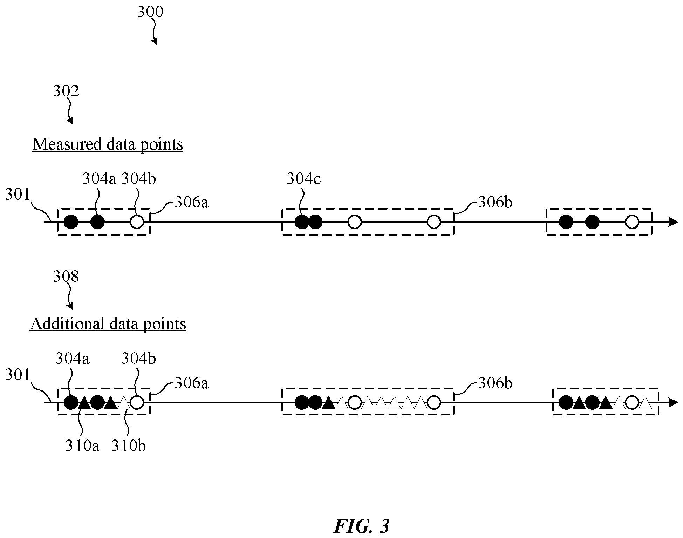

FIG. 3 shows an example of a process 300 for generating a set of additional data points 308 from a set of measured data points 302. The set of measured data points 302 may include measured data points 304 that are each collected at different time points using one or more sensors, as described herein. The set of measured data points 302 may be collected over one or more periods of time 301 and include a measured data point 304 for time points for which there was a successful cardiac measurement, while time points associated with time intervals during which there was not a successful cardiac measurements may not have a corresponding data point. Accordingly, the set of measured data points 302 may include a discontinuous set of data that has data for some time points and no data for other time points over the period of time 301.

Each measured data point is assigned a classification, which can include a first classification that indicates an occurrence of a qualified cardiac event for the corresponding time point (shown as a shaded circle in FIG. 3) or a second classification that indicates the absence of a qualified cardiac event for the correspond time point (shown as an unshaded circle in FIG. 3).

The set of additional data points 308 can be generated from the set of measured data points 302 by using the measured data points to determine a classification for each of the additional data points 310 (shown as triangles in FIG. 3), as described herein. In some cases, they system may be configured to generate additional data points 310 for time points that are within a time threshold from at least of one the measured data points 304. For example, if a time between two subsequent measured data points 304 is below the time threshold, the system may be configured to generate additional data points for time points between the two subsequent measured data points 304. This is shown for a first data cluster 306a, which may have a first measured data point 304a and a second measured data point 304b, which are separated by less than the time threshold. Accordingly, additional data points 310 may be generated for the first data cluster 306a.

By way of further example, a second data cluster 306b may have data points 304 (one of which is labeled for clarity) that are separated by less than the time threshold. However, a last measured data point of the first data cluster 306a (measured data point 304b) is separated from a first measured data point of the second data cluster 306b (measured data point 304c) by more than the time threshold. Accordingly, the system may not generate additional data points 310 between the first data cluster 306a and the second data cluster 306b. However, the system may be configured to generate the additional data points 310 within each of the data clusters 306.

In some cases, the system will generate the additional data points 310 for time points and assign a classification to each additional data point based on a classification of the nearest measured data point 304. For example, a first additional data point 310a is assigned a classification of the occurrence of a qualified cardiac event (indicated by a shaded triangle) because it corresponds to a time point that is between two measured data points that each include a classification indicating the occurrence of a qualified cardiac event. A second additional data point 310b may be assigned a classification of the absence of a qualified cardiac event (indicated by an unshaded triangle) based on the closest measured data point (measured data point 304b) having that classification.

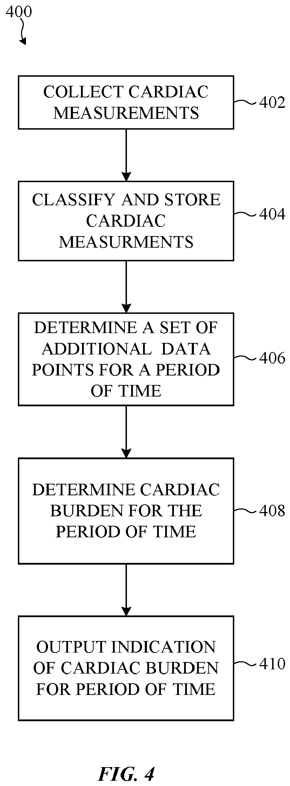

FIG. 4 shows a process flow 400 for determining cardiac burdens for different periods of time. The process 400 can be performed by the cardiac monitoring systems described herein such as the system 100. The process 400 may be used to determine one or more values of a cardiac burden of a user for multiple different periods of times, which may include the various periods of time described herein.

At operation 402 the process 400 can include collecting a series of cardiac measurements of a user which can be an example of operation 202 described with respect to FIG. 2. The measurements can be collected at a set of time points using one or more sensors, such as the sensors described herein.

At operation 404 the process 400 can include classifying and storing data corresponding to the cardiac measurements. For example, the collected cardiac measurement can be used to generate a set of measured data points, as described herein. The measured data points can be stored in a database or other suitable location and used by an electronic device to determine a cardiac burden for a user. For example, in response to initiating a cardiac burden measurement, an electronic device may retrieve measured data points.

At operation 406 the process 400 can include determining a set of additional data points for a period of time. The period of time may be hourly, daily, weekly, monthly or other defined period, such as described herein. In some cases, operation 406 can include retrieving measured data points for the period of time. For example, if the period of time is a weekly time period, operation 406 can include an electronic device retrieving the measured data points for a specific week. In other cases, if the period of time is a reoccurring time window, such as a reoccurring day of the week (e.g., cardiac burden for Mondays), operation 406 can include the electronic device retrieving the measured data points measured on Mondays.

In some cases, operation 406 can include determining whether the measured data points meet a criteria, such as a minimum amount of data over the period of time. The criteria may be set to ensure that accurate cardiac burdens are determined by ensuring that there are enough measured data points over the period of time. Additionally or alternatively, the criteria may include a distribution component which may ensure that the measured data points are from time points that are spread out over the period of time. For example, for a weekly period of time, the distribution component may require that there a defined number of measured data points collected each day. If the criteria is met, operation 406 can include determining the additional data points from the set of measured data points as described herein.

At operation 408 the process 400 can include determining a cardiac burden for the user for the period of time. In some cases, the cardiac burden can be a value that indicates an amount of time the user experiences atrial fibrillation over the predefined period of time.

At operation 410 the process 400 can include outputting an indication of the cardiac burden for the period of time. The indication of the cardiac burden can be output on a graphical interface of an electronic device and include text, graphics, animations, videos or any other suitable visual outputs. Additionally or alternatively, the indication of the cardiac burden can be output via other suitable modalities such as using audio outputs and/or haptic feedback.

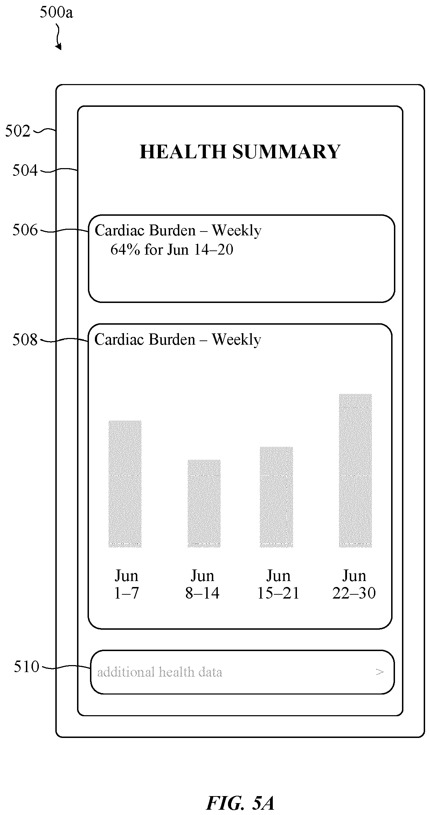

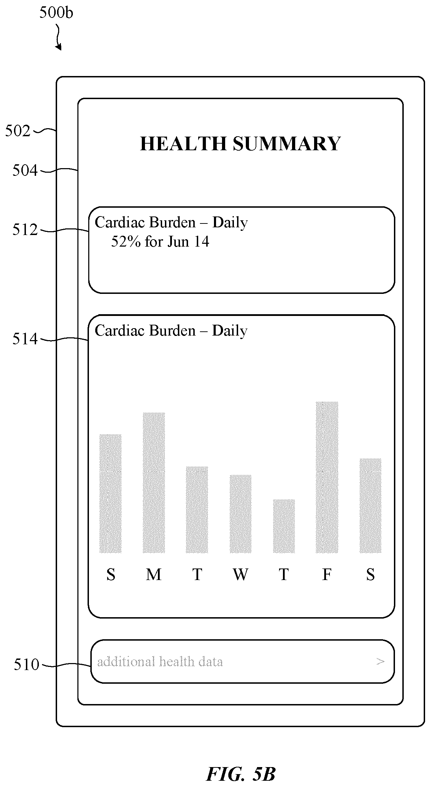

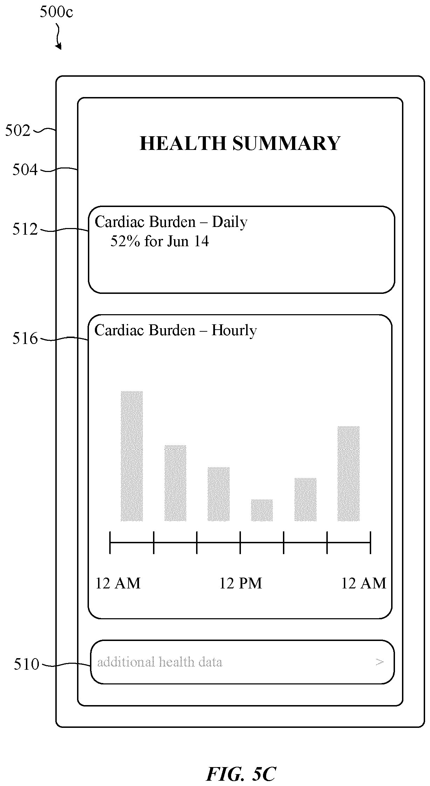

FIGs. 5A-5C show example graphical outputs 500 of cardiac burdens for different periods of times. FIG. 5A shows a first graphical output 500a of a cardiac burden for a weekly period of time; FIG. 5B shows a second graphical output 500b for a daily period of time; and FIG. 5C shows a third graphical output 500c for an hourly period of time. The graphical outputs 500 may be displayed in a user interface 504 of an electronic device, which can be an example of the electronic devices described herein.

The first graphical output 500a can include a weekly burden output 506, which indicates a user's cardiac burden for a weekly period. For example, the weekly burden output 506 may indicate an estimated percentage of a weekly period (e.g., June 14-20) that the user experienced a qualified cardiac event. In some cases, the qualified cardiac event can be detection of atrial fibrillation and the first burden output 506 can include an estimated percentage of the weekly time period that the user experienced atrial fibrillation. Additionally or alternatively, the first graphical output 500a may include a weekly burden summary 508, which may show data for multiple weeks. Accordingly, the system may track a user's cardiac burden over time and provide outputs that show how the cardiac burden changes over time. In some cases, the first graphical output 500a may also include an option to view additional data about the user's health. In some cases, this option may allow a user to enter information such as diet, exercise, stress and so on.

The second graphical output 500b can include a daily burden output 512, which indicates a user's cardiac burden for one or more daily time periods. For example, the daily burden output 512 may indicate an estimated percentage of a daily period (e.g., June 14) that the user experienced a qualified cardiac event. Additionally or alternatively, the second graphical output 500b may include a daily burden summary 514, which may show data for a reoccurring time period as described herein. The system may collect cardiac measurements over multiple weeks and combine the multi-week data to generate a re-occurring daily burden. For example, the system may combine measured data from a Mondays for multiple weeks and generate a re-occurring cardiac burden for one or more days of the week. The daily burden summary 514 shows an example where each a re-occurring burden is generated for each day of the week. In some cases, they system may need to obtain a defined amount of data prior to generating a cardiac burden for a time period, accordingly it may take multiple weeks of measuring cardiac data before a re-occurring daily burden (or other time period) can be generated.

The third graphical output 500c can include an hourly burden summary 516, which may show data for a re-occurring hourly time period. For example, each day may be broken down into hourly segments and a burden may be determined for each hourly segment using day from multiple days. Accordingly, the system may output a summary that indicates how the time of the day is associated with a cardiac burden of a user. The cardiac burdens for different periods of time may be used to identify potential causes of cardiac events or associate cardiac events with specific conditions such as dietary factors, stress, sleep and so on.

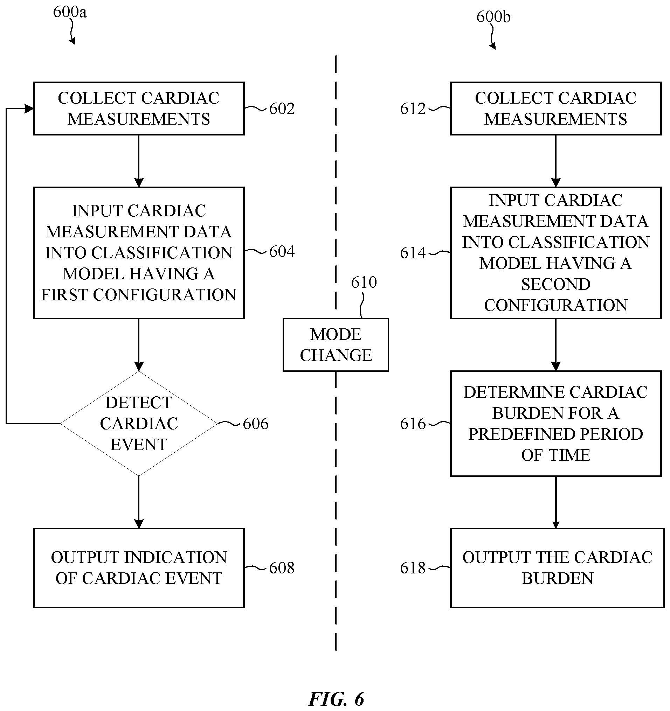

FIG. 6 shows a process flow 600 for operating a device in different cardiac monitoring modes for a given type of cardiac event. The process 600 can be performed by the cardiac monitoring systems described herein such as the system 100. An electronic device may operate in a first mode 600a that detects occurrences of cardiac events, and the process may be configured to identify whether a user experiences a specific cardiac event such as atrial fibrillation and alert the user to the occurrence of a cardiac event. The electronic device may also operate in a second mode 600b that determines a burden associated with a cardiac event such as atrial fibrillation; the second mode may track how often a particular event is occurring over a period, as described herein. In some cases, the first mode 600a may be configured to identify a cardiac event with greater specificity, which may help correctly identify is a user is experiencing a particular cardiac event. The second mode 600b, may be initiated for people who are already experiencing a particular cardiac event, and thus may be configured to identify the occurrence of a cardiac event with greater sensitivity.

The first mode 600a may be initiated by a user at an electronic device, for example by user opting into a health monitoring application. In some cases, the electronic device may prompt a user to activate the health monitoring function and initiate the first mode 600a.

At operation 602 the first mode 600a can include collecting cardiac measurements of a user which can be an example of operation 202 described with respect to FIG. 2. The measurements can be collected at a set of time points using one or more sensors, as described herein.

At operation 605 the first mode 600a can include inputting cardiac measurements into a classification model having a first configuration. The classification model may be configured to produce a first set of outputs that identifies whether a cardiac measurement (e.g., associated with a period of time) is a qualified cardiac measurements. The classification model may be a trained classification model that outputs a probability that a cardiac event occurred, as described herein. The process may qualify outputs that satisfy a first threshold (e.g., a confidence threshold as discussed previously) as an occurrence of a cardiac event. In some cases, the first threshold may be configured to provide high selectivity to identify users that have the qualified cardiac event.

At operation 606, the classification model may indicate a probability that a cardiac measurement corresponds to a qualified cardiac event and the output probability may need to be above the first threshold to characterize the cardiac measurement as corresponding to a qualified cardiac event. Accordingly, if the output from the classification model satisfies the first threshold, the cardiac measurement may be identified as a qualified cardiac event and the process may proceed to operation 606. If the output from the classification model does not satisfy the first threshold, the cardiac event may be identified as the absence of a qualified cardiac event and the process may processed to operation 602 to continue collecting cardiac measurements.

In some cases, the determination of the occurrence of qualified cardiac event may be based on the occurrence of a defined number of positive events withing a given period of time. For example, the process may include tracking a number of outputs from the classification model that satisfy a threshold, each of which may be labeled as a positive event. The process may identify the occurrence of a qualified cardiac event when the number of positive events meet the defined number of positive events. In some cases, identifying the occurrence of a cardiac event may include determining that the number of positive events occurred within a defined time period.

At operation 608 the first mode 600a can include outputting an indication of the occurrence of the qualified cardiac event to the user. For example, in cases where the qualified cardiac event is atrial fibrillation, the system may indicate that it has detected atrial fibrillation.

In some cases, in the first mode the device can collect multiple cardiac measurements and input the multiple cardiac measurements into the classification model having the first configuration. The device can be configured to output an indication of a qualified cardiac event in response to a determination that a defined number of qualified cardiac events has occurred. Accordingly, in these cases, the system may need to identify multiple qualified cardiac events before alerting a user.

At operation 610, user may switch from the first mode 600a to the second mode 600b. In some cases, the system may prompt a user to select an operating mode. For example, the system may be configured to prompt the user to switch from the first mode to the second mode in response to outputting an indication of the occurrence of the qualified cardiac event. In some cases, the system may be configured to prompt the user to switch from the first mode 600a to the second mode 600b for a number of reasons, including, but not limited to: as a response to outputting an indication of the occurrence of the qualified cardiac event; as a response to receiving new or altered health information of the user and/or the like. For example, the system may be configured to switch from the first mode 600a to the second mode 600b in response to a user indicating that they have a health condition such as atrial fibrillation.

At operation 612 the second mode 600b can include collecting cardiac measurements of a user which can be an example of operation 202 described with respect to FIG. 2. The measurements can be collected at a set of time points using one or more sensors, as described herein.

At operation 614 the second mode 600b can include inputting the cardiac measurements into a classification model having a second configuration. The classification model may be configured to produce a second set of outputs that identifies a burden for a cardiac event as described herein. The second configuration may be a second threshold for classifying a cardiac measurement as a qualified cardiac event. In some cases, the second threshold may be configured to provide high sensitivity to identify that a qualified cardiac event is occurring. In some cases, the classification model can be the same as the classification model of operation 604, but configured with a lower threshold for identifying the occurrence of a qualified cardiac event.

In some cases, the first threshold (at operation 604) can include a first probability/confidence value for identifying a qualified cardiac event and operation 614 can include a second threshold, which can be a second probability/confidence value for identifying a qualified cardiac event. The second threshold may be lower than the first threshold, which may increase the selectivity of the model at operation 614.

At operation 616 the second mode 600b can include determining a cardiac burden for one or more predefined periods of time, as described herein. At operation 618 the second mode 600b can include outputting the value of the cardiac burden, as described herein. In some cases, the modes 600a, 600b can be configured to detect and monitor atrial fibrillation. For example, the first mode 600a may operate to determine whether a user is experiencing atrial fibrillation. If atrial fibrillation is detected by the first mode and/or the user has been otherwise diagnosed as having atrial fibrillation, the second mode 600b may operate to determine a burden (e.g., amount of a predefined period of time) of the atrial fibrillation.

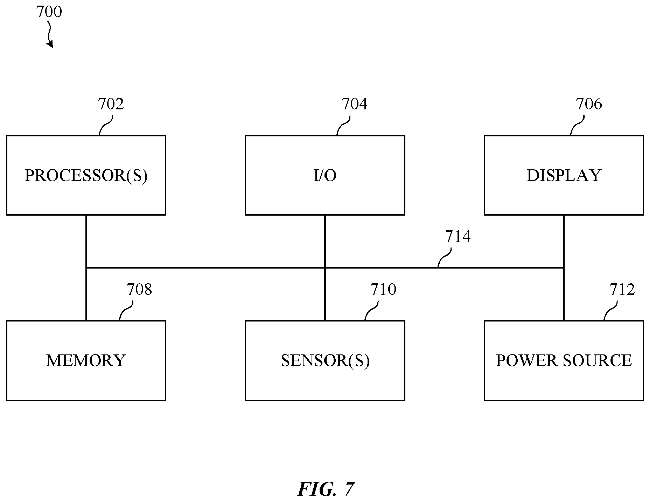

FIG. 7 is an example block diagram of a cardiac monitoring system 700, which can take the form of any of the devices as described with references to FIGs. 1-6. The cardiac monitoring system 700 can include a processor 702, an input/output (I/O) mechanism 704 (e.g., wired or wireless communications interfaces), a display 706, memory 708, sensors 710 (e.g., physiological sensors such as those described herein), and a power source 712 (e.g., a rechargeable battery). The processor 702 can control some or all of the operations of the cardiac monitoring system 700. The processor 702 can communicate, either directly or indirectly, with some or all of the components of the cardiac monitoring system 700. For example, a system bus or other communication mechanism 714 can provide communication between the processor 702, the I/O mechanism 704, the memory 708, the sensors 710, and the power source 712.

The processor 702 can be implemented as any electronic device capable of processing, receiving, or transmitting data or instructions. For example, the processor 702 can be a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitable computing element or elements. The processing unit can be programmed to perform the various aspects of the systems described herein.

It should be noted that the components of the cardiac monitoring system 700 can be controlled by multiple processors. For example, select components of the cardiac monitoring system 700 (e.g., a sensor 710) may be controlled by a first processor and other components of the cardiac monitoring system 700 (e.g., the I/O 704) may be controlled by a second processor, where the first and second processors may or may not be in communication with each other.

The I/O device 704 can transmit and/or receive data from a user or another electronic device. An I/O device can transmit electronic signals via a communications network, such as a wireless and/or wired network connection. Examples of wireless and wired network connections include, but are not limited to, cellular, Wi-Fi, Bluetooth, IR, and Ethernet connections. In some cases, the I/O device 704 can communicate with an external electronic device, such as a smartphone, electronic device, or other portable electronic device, as described here.

The cardiac monitoring system may optionally include a display 706 such as a liquid-crystal display (LCD), an organic light emitting diode (OLED) display, a light emitting diode (LED) display, or the like. If the display 706 is an LCD, the display 706 may also include a backlight component that can be controlled to provide variable levels of display brightness. If the display 706 is an OLED or LED type display, the brightness of the display 706 may be controlled by modifying the electrical signals that are provided to display elements. The display 706 may correspond to any of the displays shown or described herein.

The memory 708 can store electronic data that can be used by the cardiac monitoring system 700. For example, the memory 708 can store electrical data or content such as, for example, audio and video files, documents and applications, device settings and user preferences, timing signals, control signals, and data structures or databases. The memory 708 can be configured as any type of memory. By way of example only, the memory 708 can be implemented as random access memory, read-only memory, Flash memory, removable memory, other types of storage elements, or combinations of such devices.

The cardiac monitoring system 700 may also include one or more sensors 710 positioned almost anywhere on the cardiac monitoring system 700. The sensor(s) 710 can be configured to sense one or more types of parameters, such as but not limited to, pressure, light, touch, heat, movement, relative motion, biometric data (e.g., biological parameters), and so on. For example, the sensor(s) 710 may include a heat sensor, a position sensor, a light or optical sensor, an accelerometer, a pressure transducer, a gyroscope, a magnetometer, a health monitoring sensor, and so on. Additionally, the one or more sensors 710 can utilize any suitable sensing technology, including, but not limited to, capacitive, ultrasonic, resistive, optical, ultrasound, piezoelectric, and thermal sensing technology.

The power source 712 can be implemented with any device capable of providing energy to the cardiac monitoring system 700. For example, the power source 712 may be one or more batteries or rechargeable batteries. Additionally or alternatively, the power source 712 can be a power connector or power cord that connects the cardiac monitoring system 700 to another power source, such as a wall outlet.

As described above, one aspect of the present technology is monitoring and managing physiological conditions of a user such as cardiac events and the like. The present disclosure contemplates that in some instances this gathered data may include personal information data that uniquely identifies or can be used to contact or locate a specific person. Such personal information data can include demographic data, location-based data, telephone numbers, email addresses, Twitter IDs (or other social media aliases or handles), home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic or audiovisual outputs that are tailored to the user. Further, other uses for personal information data that benefit the user are also contemplated by the present disclosure. For instance, health and fitness data may be used to provide insights into a user's general wellness or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining the privacy and security of personal information data. Such policies should be easily accessible by users and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and revised to adhere to applicable laws and standards, including jurisdiction-specific considerations. For instance, in the US, collection of or access to certain health data may be governed by federal and/or state laws, such as the Health Insurance Portability and Accountability Act ("HIPAA"); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of determining spatial parameters, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.
Exemplary methods, devices, electronic devices, and systems are set out in the following items.
1. A method for determining a cardiac burden of a user, the method comprising:
   collecting a series of cardiac measurements at a first set of time points using one or more sensors;
   determining a set of measured data points, each measured data point comprising:
      a time value from the first set of time points; and
      a classification comprising one of an occurrence of a qualified cardiac event or absence of the qualified cardiac event, wherein the classification is selected using a cardiac measurement of the series of cardiac measurements associated with a corresponding time point from the first set of time points;
   generating a set of additional data points associated with a second set of time points different from the first set of time points, each additional data point comprising:
      a time value from the second set of time points; and
      a classification of one of the occurrence of the qualified cardiac event or the absence of the qualified cardiac event, wherein the classification is selected using one or more measured data points of the set of measured data points;
   determining a value of the cardiac burden for a predefined period of time using the set of measured data points and the set of additional data points; and
   outputting the value of the cardiac burden.
2. The method of item 1, wherein generating the set of additional data points comprises:
   determining, for each additional data point of the set of additional data points, a closest measured data point; and
   assigning, for each additional data point of the set of additional data points, the classification of the closest measured data point as the classification of the additional data point.
3. The method of item 1, further comprising selecting the second set of time points using the first set of time points, wherein each of the second set of time points is within a time threshold from at least one of the first set of time points.
4. The method of item 3, wherein:
   the time threshold is a first time threshold;
   each second time point is between two adjacent time points of the first set of time points; and
   the two adjacent time points are separated by less than a second time threshold.
5. The method of item 1, further comprising attempting, for each of a series of time intervals, to obtain a successful cardiac measurement using the one or more sensors, wherein each time point of the first set of time points is associated with a corresponding time interval of the series of time intervals in which the successful cardiac measurement was obtained.
6. The method of item 5, wherein each time point of the second set of time points is associated with a time interval of the series of time intervals in which the successful cardiac measurement was not obtained.
7. The method of item 1, wherein the qualified cardiac event is atrial fibrillation and the cardiac burden is an atrial fibrillation burden.
8. The method of item 7, wherein selecting the classification comprises inputting the cardiac measurement of the series of cardiac measurements into a classification model that is trained to detect atrial fibrillation.
9. The method of item 1, further comprising,
   determining that the set of measured data points, the set of additional data points or both meet a criteria; and
   in response to the set of measured data points, the set of additional data points or both meeting the criteria determining the value of the cardiac burden.
10. The method of item 1, wherein the one or more sensors comprises at least one of a photoplethysmography sensor or an electrocardiogram sensor.
11. A device for measuring cardiac parameters of a user, comprising:
   one or more sensors configured to collect cardiac measurements of the user; and
   a processor configured to operate in a first mode to:
      cause the one or more sensors to collect a first set of cardiac measurements;
      input each cardiac measurement of the first set of cardiac measurements as an input cardiac measurement into a classification model to produce a first set of outputs, wherein a first threshold is applied to the first set of outputs for identifying whether the input cardiac measurement is a qualified cardiac event; and
      in response to the first set of outputs meeting a first set of criteria, output an indication of an occurrence of the qualified cardiac event to the user;
   the processor configured to operate in a second mode to:
      collect a second set of cardiac measurements;
      input each of the second set of cardiac measurements as an input cardiac measurement into the classification model to produce a second set of outputs, wherein a second threshold different than the first threshold is applied to the second set of outputs for identifying whether the input cardiac measurement is the qualified cardiac event;
      determine a value of a cardiac burden for predefined period of time based on the second set of outputs; and
      output the value of the cardiac burden.
12. The device of item 11, wherein, in the first mode, the processor is further configured to:
   collect multiple first cardiac measurements;
   input the multiple first cardiac measurements into the classification model; and
   in response to the classification model producing a defined number of outputs indicating the occurrence of the qualified cardiac event, output the indication of the occurrence the qualified cardiac event to the user.
13. The device of item 12, wherein, in response to outputting an indication of the occurrence of the qualified cardiac event, the device is configured to prompt the user to switch from the first mode to the second mode.
14. The device of item 12, wherein:
   the outputs each comprise a confidence value associated with identifying the qualified cardiac event;
   the first threshold comprises a first confidence value and the second threshold comprises a second confidence value; and
   the first threshold is greater than the second threshold.
15. The device of item 11, wherein:
   the qualified cardiac event is atrial fibrillation; and
   the cardiac burden is an atrial fibrillation burden.
16. The device of item 11, further comprising:
   determining a set of measured data points, each measured data point comprising:
      a time value from the second set of cardiac measurements; and
      a classification comprising one of an occurrence of a qualified cardiac event or absence of the qualified cardiac event, wherein the classification is determined from the first set of outputs; and
   generating a set of additional data points associated with a set of time points, each additional data point comprising:
      a time value from the set of time points; and
      a classification of one of the qualified cardiac event or the absence of the qualified cardiac event, wherein the classification is selected using one or more measured data points of the set of measured data points, wherein the value of the cardiac burden is based on the set of measured data points and the set of additional data points.
17. A system for measuring cardiac parameters of a user, the system comprising:
   one or more sensors configured to collect cardiac measurements of the user; and
   a processer configured to
      collect a series of cardiac measurements at a first set of time points using the one or more sensors;
      determine a set of measured data points, each measured data point comprising:
         a time value from the first set of time points; and
         a classification comprising one of an occurrence of a qualified cardiac event or absence of the qualified cardiac event, wherein the classification is selected using a cardiac measurement of the series of cardiac measurements associated with the time value;
      generate a set of additional data points associated with a second set of time points different from the first set of time points, each additional data point comprising:
         a time value from the second set of time points; and
         a classification of one of the occurrence of the qualified cardiac event or the absence of the qualified cardiac event, wherein the classification is selected using one or more measured data points of the set of measured data points;
   determine a value of a cardiac burden for a predefined period of time using the set of measured data points and the set of additional data points; and
   output the value of the cardiac burden.
18. The system of item 17, wherein the processor is further configured to:
   determine that the set of measured data points meet a criteria; and
   the criteria comprises a minimum number of cardiac measurements over the predefined period of time.
19. The system of item 17, wherein the value of the cardiac burden indicates an amount of time the user experiences atrial fibrillation over the predefined period of time.
20. The system of item 18, wherein the predefined period of time comprises a reoccurring time window comprising one of a weekly time window, a daily time window or an hourly time window.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

## Claims

1. A method for determining a cardiac burden of a user, the method comprising:
collecting a series of cardiac measurements at a first set of time points using one or more sensors;
determining a set of measured data points, each measured data point comprising:
a time value from the first set of time points; and
a classification comprising one of an occurrence of a qualified cardiac event or absence of the qualified cardiac event, wherein the classification is selected using a cardiac measurement of the series of cardiac measurements associated with a corresponding time point from the first set of time points;
generating a set of additional data points associated with a second set of time points different from the first set of time points, each additional data point comprising:
a time value from the second set of time points; and
a classification of one of the occurrence of the qualified cardiac event or the absence of the qualified cardiac event, wherein the classification is selected using one or more measured data points of the set of measured data points;
determining a value of the cardiac burden for a predefined period of time using the set of measured data points and the set of additional data points; and
outputting the value of the cardiac burden.

2. The method of claim 1, wherein generating the set of additional data points comprises:
determining, for each additional data point of the set of additional data points, a closest measured data point; and
assigning, for each additional data point of the set of additional data points, the classification of the closest measured data point as the classification of the additional data point.

3. The method of claim 1, further comprising selecting the second set of time points using the first set of time points, wherein each of the second set of time points is within a time threshold from at least one of the first set of time points.

4. The method of claim 3, wherein:
the time threshold is a first time threshold;
each second time point is between two adjacent time points of the first set of time points; and
the two adjacent time points are separated by less than a second time threshold.

5. The method of claim 1, further comprising attempting, for each of a series of time intervals, to obtain a successful cardiac measurement using the one or more sensors, wherein each time point of the first set of time points is associated with a corresponding time interval of the series of time intervals in which the successful cardiac measurement was obtained.

6. The method of claim 5, wherein each time point of the second set of time points is associated with a time interval of the series of time intervals in which the successful cardiac measurement was not obtained.

7. The method of claim 1, wherein the qualified cardiac event is atrial fibrillation and the cardiac burden is an atrial fibrillation burden.

8. The method of claim 7, wherein selecting the classification comprises inputting the cardiac measurement of the series of cardiac measurements into a classification model that is trained to detect atrial fibrillation.

9. The method of claim 1, further comprising,
determining that the set of measured data points, the set of additional data points or both meet a criteria; and
in response to the set of measured data points, the set of additional data points or both meeting the criteria determining the value of the cardiac burden.

10. The method of claim 1, wherein the one or more sensors comprises at least one of a photoplethysmography sensor or an electrocardiogram sensor.

11. A device for measuring cardiac parameters of a user, comprising:
one or more sensors configured to collect cardiac measurements of the user; and
a processor configured to operate in a first mode to:
cause the one or more sensors to collect a first set of cardiac measurements;
input each cardiac measurement of the first set of cardiac measurements as an input cardiac measurement into a classification model to produce a first set of outputs, wherein a first threshold is applied to the first set of outputs for identifying whether the input cardiac measurement is a qualified cardiac event; and
in response to the first set of outputs meeting a first set of criteria, output an indication of an occurrence of the qualified cardiac event to the user;
the processor configured to operate in a second mode to:
collect a second set of cardiac measurements;
input each of the second set of cardiac measurements as an input cardiac measurement into the classification model to produce a second set of outputs, wherein a second threshold different than the first threshold is applied to the second set of outputs for identifying whether the input cardiac measurement is the qualified cardiac event;
determine a value of a cardiac burden for predefined period of time based on the second set of outputs; and
output the value of the cardiac burden.

12. The device of claim 11, wherein, in the first mode, the processor is further configured to:
collect multiple first cardiac measurements;
input the multiple first cardiac measurements into the classification model; and
in response to the classification model producing a defined number of outputs indicating the occurrence of the qualified cardiac event, output the indication of the occurrence the qualified cardiac event to the user.

13. The device of claim 12, wherein, in response to outputting an indication of the occurrence of the qualified cardiac event, the device is configured to prompt the user to switch from the first mode to the second mode.

14. The device of claim 12, wherein:
the outputs each comprise a confidence value associated with identifying the qualified cardiac event;
the first threshold comprises a first confidence value and the second threshold comprises a second confidence value; and
the first threshold is greater than the second threshold.

15. The device of claim 11, wherein:
the qualified cardiac event is atrial fibrillation; and
the cardiac burden is an atrial fibrillation burden.

16. The device of claim 11, further comprising:
determining a set of measured data points, each measured data point comprising:
a time value from the second set of cardiac measurements; and
a classification comprising one of an occurrence of a qualified cardiac event or absence of the qualified cardiac event, wherein the classification is determined from the first set of outputs; and
generating a set of additional data points associated with a set of time points, each additional data point comprising:
a time value from the set of time points; and
a classification of one of the qualified cardiac event or the absence of the qualified cardiac event, wherein the classification is selected using one or more measured data points of the set of measured data points, wherein the value of the cardiac burden is based on the set of measured data points and the set of additional data points.

17. A system for measuring cardiac parameters of a user, the system comprising:
one or more sensors configured to collect cardiac measurements of the user; and
a processor configured to
collect a series of cardiac measurements at a first set of time points using the one or more sensors;
determine a set of measured data points, each measured data point comprising:
a time value from the first set of time points; and
a classification comprising one of an occurrence of a qualified cardiac event or absence of the qualified cardiac event, wherein the classification is selected using a cardiac measurement of the series of cardiac measurements associated with the time value;
generate a set of additional data points associated with a second set of time points different from the first set of time points, each additional data point comprising:
a time value from the second set of time points; and
a classification of one of the occurrence of the qualified cardiac event or the absence of the qualified cardiac event, wherein the classification is selected using one or more measured data points of the set of measured data points;
determine a value of a cardiac burden for a predefined period of time using the set of measured data points and the set of additional data points; and
output the value of the cardiac burden.

18. The system of claim 17, wherein the processor is further configured to:
determine that the set of measured data points meet a criteria; and
the criteria comprises a minimum number of cardiac measurements over the predefined period of time.

19. The system of claim 17, wherein the value of the cardiac burden indicates an amount of time the user experiences atrial fibrillation over the predefined period of time.

20. The system of claim 18, wherein the predefined period of time comprises a reoccurring time window comprising one of a weekly time window, a daily time window or an hourly time window.
